# EUROPEAN PATENT APPLICATION

(11) **EP 4 458 436 A1**
(43) Date of publication of application: **06.11.2024**
(21) Application number: 22916655.8
(22) Date of filing: 26.12.2022
(51) Int. Cl.: A63B 24/00, G06Q 10/10, A63B 71/06, G06Q 30/02, G06Q 50/10, G06T 19/00

(54) **AI EXERCISE SEQUENCE PROVIDING DEVICE AND METHOD**

(30) Priority: 28.12.2021 KR 20210190369; 28.12.2021 KR 20210190370; 23.12.2022 KR 20220183461
(71) Applicant: DRAX Inc., Anyang-si, Gyeonggi-do 14086 (KR)
(72) Inventor: YOO, Seon Kyung, Seoul 08251 (KR)
(74) Representative: Mewburn Ellis LLP
(86) International application number: PCT/KR2022/021302
(87) International publication number: WO 2023/128514

(57) **Abstract**

An AI exercise sequence providing device includes: a storage unit that stores at least one muscle activated when using an exercise machine and a contribution of each of the at least one muscle; an exercise sequence generating unit configured to generate an exercise sequence by selecting a plurality of exercise machines based on a target exercise amount of a target muscle; and a measuring unit configured to calculate in real time a cumulative exercise amount applied to each of at least one muscle based on each at least one muscle that is activated when performing an exercise according to the exercise sequence and based on the contribution of each at least one muscle.

## Description

### Technical Field

The present disclosure relates to a method of providing an exercise sequence composed of a combination of exercise machines related to the muscles a user wishes to develop.

### Background Art

Weight exercise machines are provided in various forms depending on the body part to increase the muscle strength or the purpose of use, and are mainly used to train the upper and lower body using the arms or legs. A user can perform an exercise by moving a selected weight through an exercise structure of an exercise machine.

However, there is a problem in that it is difficult for users to determine the necessary exercise machine and the degree of exercise load according to their physical development status. Additionally, it is difficult to create an exercise plan with high exercise effectiveness depending on one's physical characteristics.

### Disclosure

### Technical Problem

An embodiment of the present disclosure is to provide, to a user, an exercise sequence including exercise machines related to target muscles that the user wishes to develop.

An embodiment of the present disclosure is to provide, to a user, an AI exercise sequence in which at least one of an exercise load, a number of exercises, and a type of exercise machine is modified based on the remaining exercise amount of a target muscle when the user has performed only part of a given exercise sequence due to the user's physical strength, injury, preference for other exercise machine, etc.

### Technical Solution

As an embodiment of the present disclosure, an AI exercise sequence providing device includes: a storage unit that pre-stores at least one muscle activated when a user uses an exercise machine and the contribution of each of the at least one muscle; an AI exercise sequence generating unit configured to generate an exercise sequence by selecting a plurality of exercise machines based on a target exercise amount of the target muscle; and a measuring unit configured to calculate a cumulative exercise amount applied to each of the at least one muscle based on the at least one muscle activated when performing an exercise according to the exercise sequence and the contribution of each at least one muscle.

### Advantageous Effects

In an embodiment of the present disclosure, exercise efficiency is improved by providing an exercise sequence suitable for a user based on at least one target muscle that the user wishes to develop.

In an embodiment of the present disclosure, if a user is unable to completely perform an exercise sequence due to factors that affect the user during the exercise process, such as physical strength, condition, injury, preference for exercise machines, etc., an AI exercise sequence that can replace the above exercise sequence is provided so that the user may develop a target muscle the user has intended to develop.

### Description of Drawings

FIG. 1 illustrates a smart gym system in which an AI exercise sequence providing device is used, as an embodiment of the present disclosure.
FIG. 2 illustrates an example of an exercise device implemented by a method for providing an AI exercise sequence provided in a smart gym, as an embodiment of the present disclosure.
FIG. 3 illustrates an internal configuration of exercise machines and a smart gym server in a smart gym system in which an AI exercise sequence providing method is implemented, as an embodiment of the present disclosure.
FIG. 4 illustrates an internal configuration of an AI exercise sequence providing device as an embodiment of the present disclosure.
FIG. 5 illustrates an example in which an exercise sequence is provided by an AI exercise sequence providing device, as an embodiment of the present disclosure.
FIG. 6 illustrates an embodiment of the present disclosure, showing an example of an exercise trajectory detected from an exercise machine.
FIG. 7 illustrates an example in which at least one muscle activated when performing an exercise and a cumulative exercise amount are displayed in real time on a display unit, as an embodiment of the present disclosure.

### Best Mode

As an embodiment of the present disclosure, an AI exercise sequence providing device includes a storage unit that stores at least one muscle activated when using an exercise machine and a contribution of each of the at least one muscle; an exercise sequence generating unit configured to generate an exercise sequence by selecting a plurality of exercise machines based on a target exercise amount of a target muscle; and a measuring unit configured to calculate in real time a cumulative exercise amount applied to each of at least one muscle based on each at least one muscle that is activated when performing an exercise according to the exercise sequence and based on the contribution of each at least one muscle.

As an embodiment of the present disclosure, the exercise sequence may include a plurality of exercise machines, target weight information for each of the plurality of exercise machines, and a number of times the plurality of exercise machines are used.

As an embodiment of the present disclosure, a display unit may display the at least one muscle and the cumulative exercise amount by converting, into numbers, the at least one muscle activated when performing an exercise according to the exercise sequence and the cumulative exercise amount in real time.

In an embodiment of the present disclosure, if a user fails to perform all of the exercise sequence, the measuring unit may be further configured to calculate a remaining exercise amount representing a difference between the target exercise amount of the target muscle and the cumulative exercise amount of the target muscle.

As an embodiment of the present disclosure, the AI exercise sequence generating unit may generate an AI exercise sequence based on the remaining exercise amount of the target muscle.

In an embodiment of the present disclosure, a contribution applied to the target muscle by a target weight of an exercise machine included in the AI exercise sequence is less than a construction applied to the target muscle by target weights of the plurality of exercise machines included in the exercise sequence.

As an embodiment of the present disclosure, exercise machines included in the AI exercise sequence may include aerobic exercise machines or anaerobic exercise machines.

As an embodiment of the present disclosure, the display unit may display, in numbers, at least one muscle activated as a result of performing at least a portion of the exercise sequence and the AI exercise sequence and the cumulative exercise amount of each of at least one muscle.

As an embodiment of the present disclosure, the display unit may display, on a character in numbers, the at least one activated muscle and the cumulative exercise amount of each of at least one muscle

As an embodiment of the present disclosure, the target exercise amount of the target muscle is set by reflecting an exercise rest period of the user.

### Mode for Invention

Hereinafter, embodiments of the present disclosure will be described in detail with reference to the attached drawings in order to enable those skilled in the art to easily work the present disclosure.

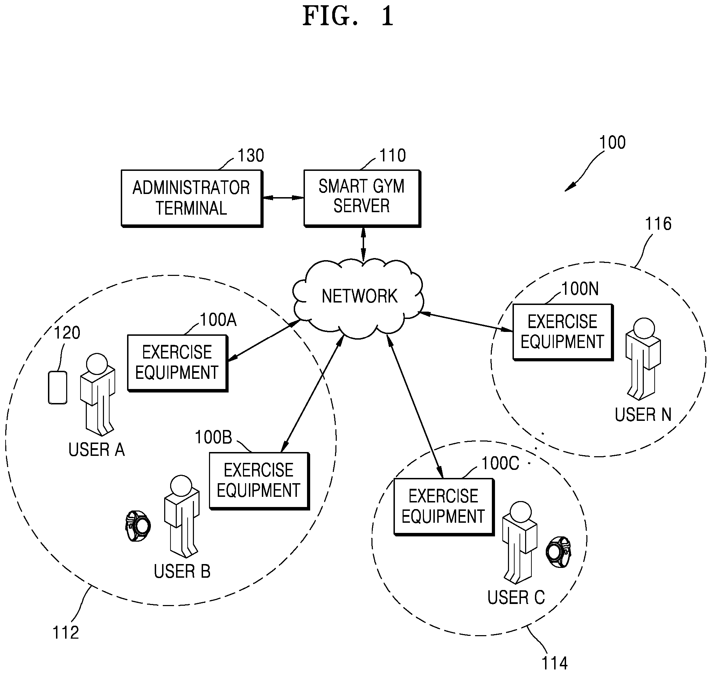

FIG. 1 illustrates a smart gym system in which an AI exercise sequence providing device is used, as an embodiment of the present disclosure.

A smart gym system 100 includes a smart gym server 110, at least one exercise machine 100a, 100b, 100c, ..., 100n, at least one user terminal 120, and an administrator terminal 130.

In FIG. 1, the smart gym server 110 is capable of communicating with a first smart gym 112, a second smart gym 114, and an nth smart gym 116, which are at physically different locations from each other, and may transmit or receive data to or from one more exercise machines 100a, 100b arranged in the first smart gym 112, at least one exercise machine 100c arranged in in the second smart gym 114, and at least one exercise machine 100n arranged in the nth smart gym 116.

In an embodiment of the present disclosure, a smart gym refers to a physical space that provides, to the smart gym server 110, exercise records of a user who has used an exercise machine, and the smart gym server 110 learns and analyzes the user's exercise records to provide exercise prescriptions that fit the needs of the user. A smart gym may be implemented as a fitness center, a gym, or a space equipped with exercise machines.

Users USER A, USER B, USER C, ..., USER N who come to a smart gym to exercise may enter the smart gym after their identity is verified. For example, a user may enter or exit after member verification by tagging the user terminal 120 to an unmanned terminal such as a kiosk at the entrance of the smart gym by using NFC (Near Field Communication) or RFID (Radio Frequency IDentification), or after member verification is completed through biometric information such as facial recognition via an unmanned terminal.

When using at least one exercise machine 100a, 100b, 100c, ..., 100n in the smart gym system 100, by using the terminal 120, the user may communicate through tagging, such as NFC or RFID, or his/her identification is verified by using the physical characteristics of the user. Once the identity of the user has been verified, the smart gym server 110 may transmit user data to an exercise machine tagged by the user. Information about the user is also referred to as user data and includes at least some or all of the gender, age, weight, height, BMI, and body fat percentage of the user.

Information about the user whose membership has been confirmed may be transmitted from a smart gym server 200 to at least one of the exercise machines 100A, 100B, 100C, ..., 100N through a network. For example, the smart gym server 200 may transmit information about the user to an exercise machine to which the user has tagged the terminal 120.

When the user tags the terminal 120 to an exercise machine, the smart gym server 200 determines that the tagged exercise machine is in an occupied state. Referring to FIG. 1, in the first smart gym 112, when a first user USER A tags the terminal 120 to a first exercise machine 100A and a second user USER B tags a terminal to the second exercise machine 100B, the smart gym server 110 determines that the first exercise machine 100A and the second exercise machine 100B are occupied.

The smart gym server 110 may provide exercise guides, exercise sequences or AI exercise sequences tailored to each user to the first user USER A and the second user USER B by using each of the at least one exercise machine 100a, 100b arranged in the smart gym 112. An exercise guide may include the speed appropriate for using each exercise machine, the range of motion of the exercise machine, and breathing methods.

The smart gym server 110 may receive exercise records of the first user USER A and the second user USER B by using each of the exercise machines 100a and 100b. Additionally, health information or log information such as the user's heart rate, blood pressure, pulse, etc. may be further received from the user terminal 120.

The smart gym server 110 may be implemented in the form of a cloud server. The smart gym server 110 is capable of integrating and managing information collected from each exercise machine in a smart gym exercise center located at different locations. For example, the smart gym server 110 may integrate and manage the details of the first user's use of exercise machine at the smart gym 112 at a first location and the details of the user's use of the exercise machine at the smart gym 114 at a second location.

As an embodiment of the present disclosure, at least one exercise machine 100a, 100b, 100c,..., 100n may be a stretching exercise machine, a weight training machine, or an aerobic exercise machine. The weight training machine includes free weight equipment and machine equipment. The at least one exercise machine 100a, 100b, 100c, ..., 100n provides a suitable exercise guide to a user through a display attached to the exercise machine or a display capable of performing wired or wireless communication with the exercise machine. For example, regarding a stretching machine, an exercise guide related to stretching is provided to a user through a smart mirror that can communicate with the stretching machine in a wired or wireless manner. However, the present disclosure is not limited thereto, and exercise guides may be provided using various output methods such as a speaker and vibration.

The at least one exercise machine 100a, 100b, 100c, ..., 100n may perform wired or wireless communication with the smart gym server 110, the user terminal 120, and the administrator terminal 130.

As an embodiment of the present disclosure, the user terminal 120 may be implemented in the form of a smartphone, smart watch, handheld device, wearable device, etc. Additionally, the user terminal 120 may have installed therein an application for using a smart gym. The user terminal 120 may receive an exercise program, exercise sequence information, or AI exercise sequence information from the smart gym server 120.

In an embodiment of the present disclosure, an exercise sequence includes an exercise program, and the exercise sequence further provides information about the order of use of each exercise machine belonging to the exercise program in addition to exercise program information.

As an embodiment of the present disclosure, the exercise program includes information such as the target weight, the number of repetitions, Reps, exercise volume, etc. of a plurality of exercise machines selected in consideration of the user' physical strength and exercise ability.

In an embodiment of the present disclosure, an AI exercise sequence refers to an exercise sequence modified from an initially given exercise sequence by newly setting, based on the remaining exercise amount of the target muscle, a target weight, the number of repetitions, Reps, the exercise volume, exercise machines, the order of use of exercise machines, etc., when the user has performed only part of a given exercise sequence and thus fails to achieve a target exercise amount of the target muscle set in the exercise sequence.

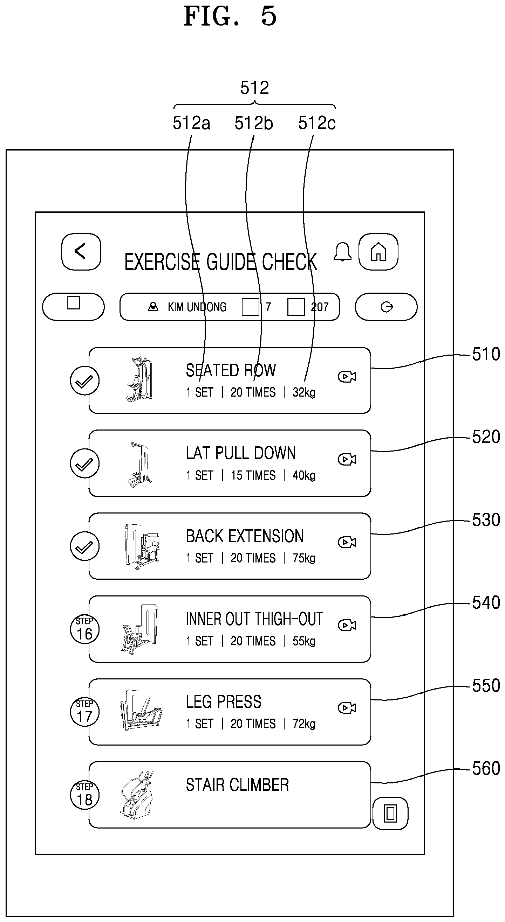

FIG. 5 illustrates an example of an exercise sequence given to a user as an embodiment of the present disclosure. FIG. 5 illustrates some of 18 exercise machines given to a user. Exercise machines 510, 520, 530 that the user has used are marked as 'exercise completed ', and exercise machines 540, 550, 560 which are not used by the user have thereon marks indicating the order of the exercise for each exercise machine 'Step 16, Step 17, Step 18'.

Referring to FIG. 5, an exercise program includes a list of exercise machines selected based on the target muscles that a user named 'Kim Undong' wants to develop, and information about Reps (512a) of the exercise machines, the number of repetitions (512b), and the target weight (512c). Examples of the list of exercise machines include seated row (510), lat pull down (520), back extension (530), inner-out thigh (540), leg press (550), and stair climber (560).

The exercise sequence refers to the order of use of exercise machines selected for user 'Kim Undong'. For example, the exercise sequence refers to using in the order of the seated row (510), the lat pull down (520), the back extension (530), the inner-out thigh (540), the leg press (550), and the stair climber (560).

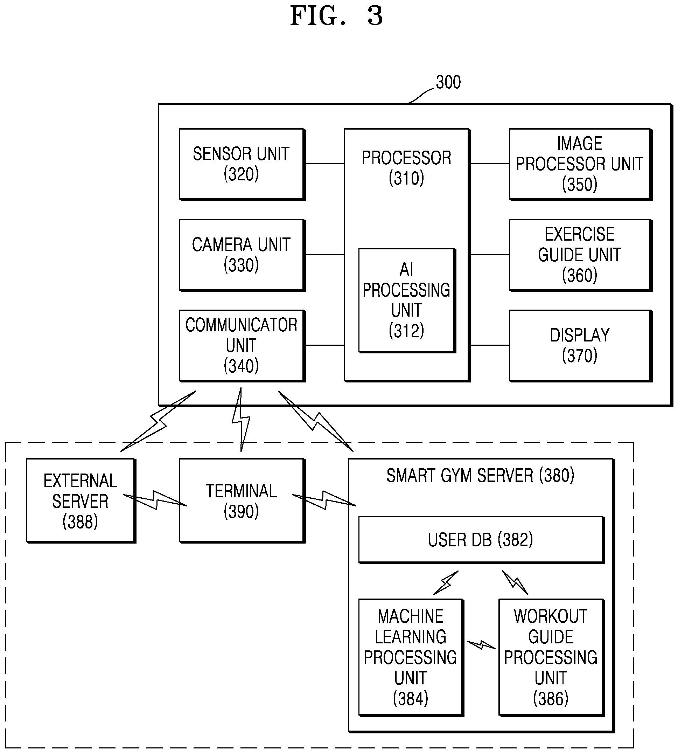

FIG. 3 illustrates an internal configuration of exercise machines and a smart gym server in a smart gym system in which an AI exercise sequence providing method is implemented, as an embodiment of the present disclosure.

As an embodiment of the present disclosure, the exercise machine 300 in a smart gym may communicate with a smart gym server 380, a user terminal 390, and an external server 388.

As an embodiment of the present disclosure, the exercise machine 300 includes a processor 310, a sensing unit 320, a communication unit 340, an exercise guide unit 360, and a display 370. In addition, the exercise machine 300 may further include a camera unit 330 and an image processing unit 350. The processor 310 may further include an AI processing unit 312, if necessary.

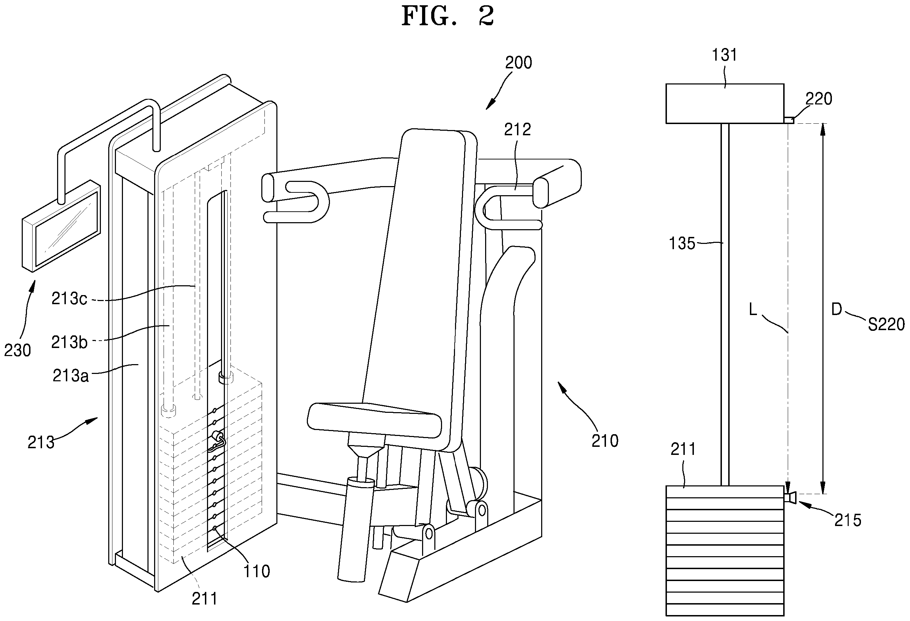

The exercise machine 300 will be described with reference to FIGS. 2 and 3. FIG. 2 illustrates a shoulder press 200, which is an example of exercise machine.

The sensing unit 220 may be installed on a frame structure 213 of an exercise body 210. The frame structure 213 includes a base frame 213a, a guide rail 213b, and a connection line 213c. The sensing unit 220 irradiates a laser beam toward a pin structure 215, receives a reflected laser beam, and measures a distance D from the sensing unit 220 to the pin structure 215 in real time or in preset time units (t) (S220). Accordingly, the sensing unit 220 may detect at least one of a position, moving speed, and moving direction of a weight member 211 selected by the pin structure 215 in real time. In addition, when the user pushes a handle 212 of the exercise machine 200 and a weight plate moves, the sensing unit 220 may measure the distance D (S220) to the pin structure 215 inserted into the weight plate, thereby detecting a movement trajectory based on the distance.

The communication unit 340 may receive a user input through a display unit 230 or transmit and receive user data from a user DB 382 of the smart gym server 380. The communication unit 340 is also capable of communicating with the external server 388.

The exercise guide unit 360 may provide, to a user, information such as user data received from the smart gym server 380, a target weight of an exercise machine, a movement speed guide of the exercise machine, a breathing guide for using the exercise machine, an exercise sequence, and AI exercise sequence. The exercise guide unit 360 displays the exercise sequence or AI exercise sequence received from the smart gym server 380 on the display 370.

As an embodiment of the present disclosure, the smart gym server 380 may include the user DB 382, a machine learning processing unit 384, and an exercise guide processing unit 386. The user DB 382 stores and manages user data. User data includes the gender, age, weight, height, BMI, and body fat percentage of the user. The machine learning processing unit 384 analyzes, accumulates, learns, and processes big data including user data obtained from the user DB 382 and exercise records of exercise machines used by users using a smart gym. The exercise guide processing unit 386 processes exercise programs, exercise sequences, and AI exercise sequence information to be provided to the user. In addition, movement speed guides for exercise machines, breathing guides for when using exercise machines, etc. may be further processed.

The smart gym server 380 may calculate the estimated strength value of a specific muscle strength as shown in Equation 1 based on user data. An example of the specific muscle strength may be grip strength. estimated muscle strength = A*gender + B*age + C*body fat percentage + D*BMI + E

In Equation 1, A, B, C, D, and E represent preset values.

The smart gym server 380 stores percentile information about the relative grip strength calculated through Equation 1. Table 1 shows the percentiles of adult relative grip strength for men, and Table 2 shows the percentiles for adult relative grip strength for women.

**[Table 1]**

| percentile | age | | | | |
|---|---|---|---|---|---|
| | 19-29 | 30-39 | 40-49 | 50-59 | 60-69 |
| 90 | **.* | ... | | | **.* |
| ... | | | | | |
| 50 | 57.2 | | 56.1 | **.* | **.* |
| 40 | **.* | 55.9 | **.* | 53.8 | 49.2 |
| ... | | | | | |
| 10 | **.* | ... | | | **.* |

**[Table 2]**

| percentile | age | | | | |
|---|---|---|---|---|---|
| | 19-29 | 30-39 | 40-49 | 50-59 | 60-69 |
| 90 | **.* | ... | | | 47.5 |
| ... | | | | | |
| 50 | **.* | 46.3 | 43.8 | **.* | **.* |
| 40 | 40.3 | **.* | **.* | **.* | 34.1 |
| ... | | | | | |
| 10 | 31.7 | ... | | | **.* |

In addition, the smart gym server 380 holds a mapping table that stores PMW_{estimated} percentile information generated based on personal maximum weight (PMW) data obtained from the population for each exercise machine, as shown in Table 3. Table 3 shows PMW_{estimated} percentile information for a leg extension exercise machine.

**[Table 3]**

| PMW_{estimated} percentile value | Population PMW_{estimated} (kg) |
|---|---|
| PMW90 | 103.3 |
| ... | ... |
| PMW50 | 67.3 |
| PMW40 | 66.5 |
| ... | ... |
| PMW10 | 24.6 |

In an embodiment of the present disclosure, a personal maximum weight (PMW) refers to the strength that a person can exert against the resistance of a weight with maximum effort. Examples of the PMW include 1RM, 4RM, etc. The PMW_{estimated} represents an estimated value of the user's PMW based on the estimated muscle strength value calculated through Equation 1. The smart gym server 380 may determine the PMW_{estimated} for each exercise machine. The smart gym server 380 determines a PMW_{estimated} of a exercise machine to be used by a user based on the estimated muscle strength value and PMW_{estimated} percentile information. In addition, based on the PMW_{estimated}, an initial target weight of the exercise machine to be used by the user is automatically set.

For example, if the estimated strength value of a 32-year-old man is 55.9 and the percentile value to which the estimated strength value belongs is 40, the corresponding estimated PMW_{estimated} percentile value of 40, or 66.5kg, is estimated as a leg extension PMW of a 32-year-old man. As an embodiment of the present disclosure, the smart gym server 380 pre-stores PMW_{estimated} percentile information for each exercise machine as shown in Table 1.

The exercise guide processing unit 386 sets an initial target weight of each exercise machine based on the PMW_{estimated}. In this case, the exercise guide processing unit 386 may set the initial target weight according to the exercise intensity or exercise purpose. The exercise intensity may be determined according to the exercise volume to be provided in the exercise sequence of a preset period. For example, it is assumed that a preset unit period is one week. When an exercise volume to be given to each muscle for a week is determined, the exercise guide processing unit 386 may set an exercise intensity according to the rest period for each muscle and the cumulative exercise volume during a unit period. The exercise volume refers to a product of the weight, the number of repetitions, and sets.

The exercise guide processing unit 386 may set the weight as a% of the PMW_{estimated} when the exercise intensity is low intensity, b% of the PMW_{estimated} when the exercise intensity is medium intensity, and c% of the PMW_{estimated} when the exercise intensity is high intensity. For example, a may be set to 20 to 40, b may be set to 40 to 60, and c may be set to 60 to 80. However, it should be noted that this is only an example and various modifications are possible.

The exercise guide processing unit 386 may provide the initial target weight of the exercise machine to be used by the user to the exercise guide unit 360 of the exercise machine 300, and the exercise guide unit 360 may display information, on the display 370, the initial target weight of the exercise machine (FIG. 5, 512c) to be used by the user.

As another embodiment of the present disclosure, the smart gym server 380 may predict a PMWₚₑᵣₛₒₙₐₗ, which is a maximum muscle strength value in which an objectification index for each user is reflected. And, the smart gym server 380 may update the target weight of the exercise machine (FIG. 5, 512c) based on the predicted PMWₚₑᵣₛₒₙₐₗ. In detail, the smart gym server 380 sets the initial target weight of the exercise machine based on PMW_{estimated}, and when the individual user's objectification index is obtained during a preset period of time, the smart gym server 380 reflects the individual user's objectification index to predict the PMWₚₑᵣₛₒₙₐₗ. Afterwards, the target weight of the exercise machine is updated based on the PMWₚₑᵣₛₒₙₐₗ. The exercise guide processing unit 386 may display the updated target weight based on the PMWₚₑᵣₛₒₙₐₗ on the exercise machine to be used by the user.

An example of the objectification index is exercise records, and exercise records include the weight of exercise machine, the number of repetitions (Reps), the number of sets, an exercise trajectory, a movement speed, and regularity of the number of repetitions (Reps) per set (Reps), which are identified when using an exercise machine.

The machine learning processing unit 384 learns and processes the objectification index including the exercise records of exercise machines used by a user in a smart gym. The machine learning processing unit 384 may update the PMW_{estimated} to a PMWₚₑᵣₛₒₙₐₗ based on the objectification index of the exercise machine used by the user in the smart gym for a certain period of time. The machine learning processing unit 384 may update the PMWₚₑᵣₛₒₙₐₗ to a value greater than the PMW_{estimated} when the objectification index is greater than or equal to a first reference value, and update the PMWₚₑᵣₛₒₙₐₗ to a value smaller than the PMW_{estimated} when the objectification index is less than or equal to a second reference value.

Accordingly, even when the PMW_{estimated} of the first user and the second user who have the same gender, age, weight, height, BMI, and body fat percentage are estimated to be the same, by further reflecting the individual objectification index, including exercise records, etc. of the first user and the second user, the machine learning processing unit 384 may learn and predict PMWₚₑᵣₛₒₙₐₗ suitable for the first user and the second user, for each exercise machine used by the first user and the second user.

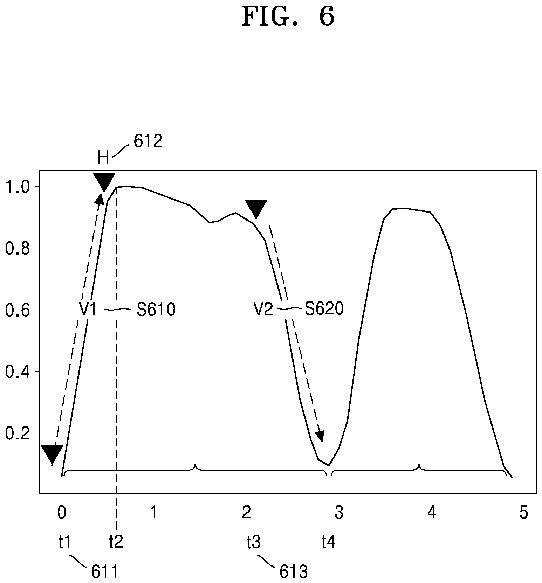

An example of generating an objectification index by the machine learning processing unit 384 is as follows. Referring to FIG. 6, by using at least one of an ascending start point 611, a descending start point 613, an ascending section speed V1 S610, a descending section speed V2 S620, the ascending section average speed, the descending section average speed, and a height H 612, identified from an exercise trajectory detected while the user was using a shoulder press, the completeness of the exercise trajectory may be determined, and the degree of completeness of the exercise trajectory may be converted to numbers and used as an objectification index. For example, if the ascending start time is between 0 and tₐ seconds, it is determined to be appropriate, and if the ascending start deviates from tₐ, it is determined to be late. In addition, ratings of high, medium, and low or points of scores given in increments of 1 point to 10 points may be given to the completeness based on a standard that is preset for the appropriate ascending start time. Likewise, if the ascending section speed V1 is between Va and Vb according to the preset standard, it is determined to be appropriate; if the ascending section speed V1 exceeds Vb, it is determined to be fast; if the ascending section speed V1 is less than Va, it is determined to be slow; and the determination results of appropriate, fast, and slow are rated as high, medium, and low, respectively, or given points of scores in increments of 1 point to 10 points. As described above, the machine learning processing unit 384 may generate an objectification index from the exercise records of the user.

As another embodiment of the present disclosure, when the number of repetitions (Reps) is used as an objectification index, the machine learning processing unit 384 may determine the completeness of the number of repetitions (Reps) based on the regularity in the respective exercise trajectories of the total number of repetitions constituting one set and the period of time taken to perform all of the total number of repetitions constituting the one set, and convert the degree of the completeness of the number of repetitions (Reps) into numbers and use the same as an objectification index.

As an embodiment of the present disclosure, the smart gym server 380 pre-stores information about at least one muscle activated when using an exercise machine and the contribution of each of the at least one muscle, as shown in Tables 4 to 5.

**[Table 4]**

| **exercis e machin e** | **joint movement** | **exercise target muscles (main muscle)** | **muscle** | |
|---|---|---|---|---|
| leg extensi on | knee extension | quadriceps | major muscles (segmentation) | contribut ion |
| | | | rectus femoris | 20% |
| | | | vastus lateralis oblique | 80% |
| | | | vastus medialis oblique | |
| | | | vastus intermedius | |

**[Table 5]**

| **exercis e machin e** | **joint movement** | **exercise target muscles (main muscle)** | **muscle** | |
|---|---|---|---|---|
| seated leg curl | knee flexion | hamstring | major muscles (segmentation) | contribut |
| | | | | ion |
| | | | biceps femoris | 60% |
| | | | semitendinosus | 15% |
| | | | semimembranosus | 15% |
| | | | accessory muscles | contribut ion |
| | | | gastrocnemius | 10% |

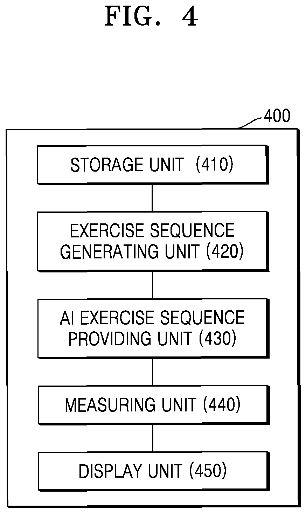

FIG. 4 illustrates an internal configuration of an AI exercise sequence providing device 400 as an embodiment of the present disclosure. The AI exercise sequence providing device 400 may also be implemented in the form of a smart gym server or cloud server.

A storage unit 410 stores at least one muscle activated when using an exercise machine and the contribution of each muscle, as shown in Tables 4 and 5. In addition, the contribution of muscles and muscles activated for each joint and range of motion used when using an exercise machine is stored.

An example of a leg extension exercise machine will be described.

Referring to Table 4, the storage unit 410 stores the quadriceps femoris as the main muscle of the leg extension exercise machine. In addition, the storage unit 410 stores information indicating that the initial section where the knee is straightened is a section where the angle of the user's knee joint is 90-81°, and the contribution of the rectus femoris is 20%, and in a section where the angle of the knee joint is 80-5°, which is a process of straightening the knee, the contribution of the vastus lateralis oblique, vastus medialis oblique, and vastus intermedius is 80%, and a section where the knee is most straightened is a section where the knee joint angle is 10-5°, and among the vastus lateralis oblique, vastus medialis oblique, and vastus intermedius, vastus medialis oblique is most active.

As an example of another exercise machine, an example of a seated dread curl exercise machine will be described.

Referring to Table 5, the storage unit 410 stores the hamstrings as the main muscle of a seated dread curl exercise machine. In addition, the storage unit 410 stores information indicating that the initial section where the knee is bent is a section where the user's knee joint is at an angle of 0-4°, and the contribution of the gastrocnemius muscle is 10%, and in a section where the knee joint angle is 5-90°, which is a section corresponding to a process of bending the knee, the contribution of the biceps femoris is 60%, the contribution of the semitendinosus muscle is 15%, and the contribution of the semimembranosus muscle is 15%. In addition, information indicating that, among the knee bending sections, with the knee joint angle of 50-90°, the biceps femoris muscle is most active is previously stored.

The exercise sequence generating unit 420 generates an exercise sequence by selecting a plurality of exercise machines based on a target exercise amount of a target muscle. As an embodiment of the present disclosure, when the target muscle is the hamstring, the exercise sequence generating unit 420 selects an exercise machine by further referring to joint information used to activate the hamstring. Examples of the selected exercise machine include Romanian deadlifts, stiff deadlifts, seated drag curls, and lying leg curls.

The exercise sequence generating unit 420 may set the target exercise amount of the target muscle based on the user's specific muscle strength value estimated based on at least some of the user's gender, age, weight, height, BMI, and body fat percentage. An example of a specific strength value is grip strength. Based on the estimated muscle strength value calculated using Equation 1, the initial target weight of the selected exercise machine may be automatically set according to a preset ratio.

The exercise sequence generating unit 420 may set the target exercise amount of the target muscle based on PMWₚₑᵣₛₒₙₐₗ identified based on a weight of an exercise machine, the number of repetitions (Reps), the number of sets, an exercise trajectory, a movement speed of the exercise machine, and the regularity of the number of repetitions (Reps) set of the exercise machine, which are identified while the user is using the exercise machine during a preset period of time. A target weight updated based on the PMWₚₑᵣₛₒₙₐₗ may be provided to the user.

The exercise sequence generating unit 420 may set the number of repetitions and Reps of each exercise machine according to the exercise intensity preset by the user.

For example, it is assumed that the target exercise amount for the hamstrings is 80 kg. The target exercise amount is calculated as weight * number of repetitions * set * contribution. When the exercise sequence generating unit 420 selects the seated drag curl as the first exercise machine, the amount of exercise is calculated based on the initial target weight * number of repetitions * set * contribution of the seated drag curl. For example, when the initial target weight for seated drag curls is 20 kg, 10 repetitions, 1 set, and the contribution is 60%, the exercise amount is calculated as 20kg*10*1*60%=12. If the initial target weight of a lying leg curl, another exercise machine related to the hamstrings, is 30 kg, 10 repetitions, 1 set, and 40 % of contribution, the exercise amount is calculated as 30kg*10*1*40%=12.

When the target muscle is the hamstring and the target exercise amount is 80 kg, the exercise sequence generating unit 420 selects at least one exercise machine from a list of exercise machines that strengthen the hamstring, sets the target weight, the number of repetitions, and the number of sets by using contribution information of each of the selected at least one exercise machine. The list of exercise machines that strengthen the hamstrings may include, for example, Romanian deadlifts, stiff deadlifts, seated drag curls, and lying leg curls.

Although the above description is based on one target muscle, for which the exercise sequence generating unit 420 generates an exercise sequence, the exercise sequence generating unit 420 may generate an exercise sequence for activating at least one target muscle. For example, to activate the hamstrings, muscle gluteus medius, and erector spinae muscles, at least one exercise machine may be selected from a list of exercise machines related to each muscle or a list of exercise machines to train joints related to each muscle.

The measuring unit 440 calculates in real time a cumulative exercise amount applied to each at least one machine based on the at least one muscle activated when performing an exercise according to the exercise sequence generated by the exercise sequence generating unit 420 and based on the contribution of each of at least one muscle. The display unit 450 displays the at least one muscle activated when performing an exercise according to the exercise sequence and the cumulative exercise amount in numbers in real time.

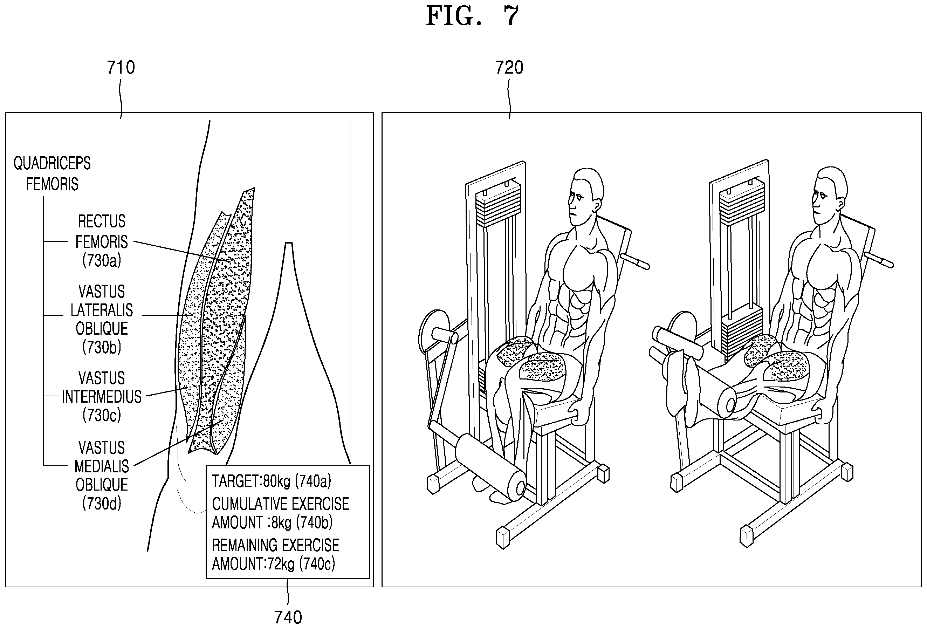

This will be described with reference to FIG. 7. When a user exercises a leg extension 700 according to the initial target weight of 20 kg, the number of repetitions of 20, and one Rep, the measuring unit 440 calculates in real time a cumulative exercise amount of each of at least one muscle activated when the user performs an exercise.

For example, in the case of 20 kg and 2 repetitions, the measuring unit 440 calculates the cumulative exercise amount of the rectus femoris muscle as 20kg*2*20%=8kg (740b) in a section where the user's knee joint angle is 90-81°. In this case, the 'rectus femoris 730a', which is the activated muscle, is displayed in color or shade or the like on a display unit 710. The display unit 710 may display activated muscles two-dimensionally or three-dimensionally. The user may intuitively understand the currently activated muscle by viewing the display unit 710. In the section when the user's knee joint angle is 80-5°, the cumulative exercise amount of vastus lateralis, vastus medialis, and vastus intermedius is calculated as 20kg*2*80%. Additionally, if the user fails to perform all of the exercise sequence, the measuring unit 440 calculates the remaining exercise amount, which represents a difference between the target exercise amount of the target muscle and the cumulative exercise amount of the target muscle. In this case, referring to FIG. 7, the display unit 710 may display an exercise progress status 740. The exercise progress status 740 includes at least one of the target exercise amount 740a, the cumulative exercise amount 740b, and the remaining exercise amount 740c. The display unit 710 displays at least one muscle (730a, 730b, 730c, 730d) activated when performing an exercise and the cumulative exercise amount 740b in real time. In another embodiment of the present disclosure, the display unit 720 may display at least one activated muscle and the cumulative exercise amount of each of the at least one muscle on a character. In addition, the exercise progress status 740 may be further displayed on a character, so that at least one muscle activated through an exercise and the cumulative exercise amount of each of the at least one muscle may be displayed in numerical form on the character.

As an embodiment of the present disclosure, the AI exercise sequence providing device 400 further includes an AI exercise sequence generating unit 430. If the user performs only part of a given exercise sequence and there is remaining exercise for each of at least one target muscle, the AI exercise sequence generating unit 430 provides an AI exercise sequence including changes to the exercise load, the number of times of exercises, Reps, type of exercise machines, etc. to the user. For example, if a user is injured while using a certain exercise machine, or if a certain joint is bad, an AI exercise sequence is provided by reflecting the user's physical strength, etc.

The AI exercise sequence generation unit 430 provides an AI exercise sequence that is newly generated by selecting exercise machines that can replace those exercise machines with remaining exercise among the exercise machines included in the exercise sequence identified by the measuring unit 440 by the user. Here, an example of using the hamstrings as the target muscle is assumed. If the user has difficulty performing the seated drag curl included in the exercise sequence, the AI exercise sequence generating unit 430 provides an AI exercise sequence including alternative exercise machine such as Romanian deadlift, stiff deadlift, and lying leg curl instead of the seated drag curl.

Exercise machines included in the AI exercise sequence include aerobic exercise machine or anaerobic exercise machine. Additionally, the AI exercise sequence may further include stretching related to the target muscle. The display unit 450 displays the AI exercise sequence provided by the AI exercise sequence generating unit 430, and also displays, in numbers, at least one muscle activated as a result of performing at least a portion of the exercise sequence and the AI exercise sequence and the cumulative exercise amount of each of at least one muscle. For example, when performing part of an exercise program of seated drag curl included in an exercise sequence and then performing the lying leg curl exercise program included in the AI exercise sequence, the cumulative exercise amounts of the seated drag curl and the lying leg curl may be summed and displayed.

FIG. 5 shows an example in which an AI exercise sequence providing device provides an exercise sequence or an AI exercise sequence to a user terminal or a smart mirror provided in a smart gym, as an embodiment of the present disclosure.

FIG. 5 shows some exercise machine among 18 exercise machines selected for a plurality of target muscles. The exercise machines 510, 520, 530 that a user has used are marked as activated and 'exercise completed', and the exercise machines 540, 550, 560 which are not used by the user have thereon marks indicating the order of the exercise for each exercise machine 'Step 16, Step 17, Step 18'.

Referring to FIG. 5, the user uses the exercise machines in the order of the seated row (510), lat pull down (520), back extension (530), inner-out thigh (540), leg press (550), and stair climber (560) according to the exercise sequence.

FIG. 6 illustrates an embodiment of the present disclosure, showing an example of an exercise trajectory detected by an exercise machine. By using at least one of an ascending start point 611, a descending start point 613, an ascending section speed V1 S610, a descending section speed V2 S620, the ascending section average speed, the descending section average speed, and a height H 612, identified from an exercise trajectory detected while the user was using a shoulder press, the machine learning processing unit 384 may determine the completeness of the exercise trajectory, and convert the degree of completeness of the exercise trajectory to numbers as an objectification index.

In addition, the exercise sequence generating unit 420 may detect certain muscles that are incorrectly used when using an exercise machine, by using exercise trajectory information obtained when the user is using the exercise machine and information about muscles activated for each range of motion of joints when using exercise machines previously stored in a storage unit.

For example, it is assumed that an exercise trajectory obtained when the user is using a leg extension exercise machine meets the preset standard in the initial section where the knee is extended, but deviates from the preset exercise trajectory in a section where the knee is extended.

The exercise sequence generating unit 420 determines that it is difficult for the user to extend the knee, and creates an exercise program in the exercise sequence generating unit 420 to exercise only the range of motion corresponding to the initial section in which the knee is straightened when using the leg extension exercise machine. In other words, only the rectus femoris muscle, which is activated in the initial section of knee extension, is activated. In addition, it may be detected that there is a problem with at least one of the user's vastus lateralis, vastus medialis, and vastus intermedius muscles or that the user's method of using the exercise machine is incorrect.

Methods according to embodiments of the present disclosure may be implemented in the form of program instructions that can be executed through various computer means and recorded on a computer-readable medium. The computer-readable medium may include program instructions, data files, data structures, etc., singly or in combination. The program instructions recorded on the medium may be those specifically designed and configured for the present disclosure, or may be known and usable by those skilled in the art of computer software.

As described above, although the present disclosure has been described with reference to limited embodiments and drawings, the present disclosure is not limited to the above embodiments, and various modifications and variations can be made from these descriptions by those skilled in the art.

## Claims

1. An AI exercise sequence providing device comprising:
a storage unit that stores at least one muscle activated when using an exercise machine and a contribution of each of the at least one muscle; an exercise sequence generating unit configured to generate an exercise sequence by selecting a plurality of exercise machines based on a target exercise amount of a target muscle; and
a measuring unit configured to calculate in real time a cumulative exercise amount applied to each of at least one muscle based on each at least one muscle that is activated when performing an exercise according to the exercise sequence and based on the contribution of each at least one muscle.

2. The AI exercise sequence providing device of claim 1, wherein the exercise sequence comprises a plurality of exercise machines, target weight information for each of the plurality of exercise machines, and a number of times the plurality of exercise machines are used.

3. The AI exercise sequence providing device of claim 1, further comprising a display unit that displays the at least one muscle and the cumulative exercise amount by converting, into numbers, the at least one muscle activated when performing an exercise according to the exercise sequence and the cumulative exercise amount in real time.

4. The AI exercise sequence providing device of claim 1, wherein, if a user fails to perform all of the exercise sequence, the measuring unit is further configured to calculate a remaining exercise amount representing a difference between the target exercise amount of the target muscle and the cumulative exercise amount of the target muscle.

5. The AI exercise sequence providing device of claim 4, further comprising an AI exercise sequence generating unit configured to generate an AI exercise sequence based on the remaining exercise amount of the target muscle.

6. The AI exercise sequence providing device of claim 5, further comprising a display unit that displays, in numbers, at least one muscle activated as a result of performing at least a portion of the exercise sequence and the AI exercise sequence and the cumulative exercise amount of each of at least one muscle.

7. The AI exercise sequence providing device of claim 6, wherein the display unit displays, on a character in numbers, the at least one activated muscle and the cumulative exercise amount of each of at least one muscle.

8. The AI exercise sequence providing device of claim 5, wherein exercise machines included in the AI exercise sequence comprise aerobic exercise machines or anaerobic exercise machines.

9. The AI exercise sequence providing device of claim 1, wherein the AI exercise sequence generating unit further includes, in the exercise sequence, stretching related to the target muscle.

10. The AI exercise sequence providing device of claim 1, wherein the target exercise amount of the target muscle is set based on a certain muscle strength value of the user, which is estimated based on at least some of a gender, an age, a weight, a height, a BMI, and a body fat percentage of the user.

11. The AI exercise sequence providing device of claim 1, wherein the target exercise amount of the target muscle is set based on a PMWₚₑᵣₛₒₙₐₗ which is identified based the weight of exercise machine, the number of repetitions (Reps), the number of sets, an exercise trajectory, a movement speed, and regularity of the number of repetitions (Reps) per set (Reps), which are identified when the user is using an exercise machine during a preset period of time.

12. An AI exercise sequence providing method comprising:
generating, by an exercise sequence generating unit, an exercise sequence by selecting a plurality of exercise machines based on a target exercise amount of a target muscle; and
calculating, by a measuring unit, a cumulative exercise amount applied to each at least one machine based on the at least one muscle activated when a user performs an exercise according to the exercise sequence and based on a contribution of each of at least one muscle; and
displaying, by a display unit, the at least one muscle and the cumulative exercise amount by converting, into numbers, the at least one muscle activated when performing an exercise according to the exercise sequence and the cumulative exercise amount in real time.

13. The AI exercise sequence providing method of claim 12, wherein the exercise sequence comprises a plurality of exercise machines, target weight information for each of the plurality of exercise machines, and a number of times the plurality of exercise machines are used.

14. The AI exercise sequence providing method of claim 12, further comprising calculating, by the measuring unit, a remaining exercise amount representing a difference between the target exercise amount of the target muscle and the cumulative exercise amount of the target muscle, if the user fails to perform all of the exercise sequence, and generating, by the AI exercise sequence generating unit, an AI exercise sequence based on the remaining exercise amount of the target muscle.

15. A computer-readable recording medium, wherein the computer-readable recording medium stores instructions that cause a computing device to perform operations of an AI exercise sequence providing method, the method comprising:
generating, by an exercise sequence generating unit, an exercise sequence by selecting a plurality of exercise machines based on a target exercise amount of a target muscle; and
calculating, by a measuring unit, a cumulative exercise amount applied to each at least one machine based on the at least one muscle activated when a user performs an exercise according to the exercise sequence and based on a contribution of each of at least one muscle; and
displaying, by a display unit, the at least one muscle and the cumulative exercise amount by converting, into numbers, the at least one muscle activated when performing an exercise according to the exercise sequence and the cumulative exercise amount in real time.
